Europäisches Patentamt

European Patent Office

Office européen des brevets

(19)

(11) Veröffentlichungsnummer: **0 280 077 B1**

# (12) EUROPÄISCHE PATENTSCHRIFT

(45) Veröffentlichungstag der Patentschrift: 08.01.92

(51) Int. Cl.5: **A61K 7/16, C01B 25/32**

(21) Anmeldenummer: **88101513.5**

(22) Anmeldetag: **03.02.88**

(54) **Zahnpasten sowie Putzkörper für Zahnpasten auf Basis Dicalciumphosphat-dihydrat und ein Verfahren zur Herstellung solcher Putzkörper.**

(30) Priorität: **24.02.87 DE 3705845**

(43) Veröffentlichungstag der Anmeldung:
**31.08.88 Patentblatt 88/35**

(45) Bekanntmachung des Hinweises auf die
Patenterteilung:
**08.01.92 Patentblatt 92/02**

(84) Benannte Vertragsstaaten:
**BE DE FR GB**

(56) Entgegenhaltungen:
**EP-A- 0 017 784**
**EP-A- 0 040 938**
**DE-A- 2 648 061**

(73) Patentinhaber: **HOECHST AKTIENGESELL-
SCHAFT**
**Postfach 80 03 20**
**W-6230 Frankfurt am Main 80(DE)**

(72) Erfinder: **Dany, Franz-Josef, Dr.**
**Heddinghovener Strasse 47**
**W-5042 Erftstadt(DE)**
Erfinder: **Klassen, Horst, Dr.**
**Hellenthaler Weg 11**
**W-5042 Erftstadt(DE)**
Erfinder: **Adrian, Renate**
**Grosse Ölbruchstrasse 29**
**W-5030 Hürth(DE)**
Erfinder: **Prell, Hedwig**
**Dr.-Krauss-Strasse 9**
**W-5030 Hürth(DE)**
Erfinder: **Kalteyer, Gerhard**
**Laurentiusstrasse 8**
**W-5042 Erftstadt(DE)**

## Beschreibung

Seit Jahzehnten werden Dicalciumphosphat-dihydrat (DCP-D) und wasserfreies Dicalciumphosphat (DCP-A) als Putzkörper in Zahnpasten eingesetzt. Neben den remineralsierenden Eigenschaften von DCP-D/DCP-A - sie enthalten praktisch dieselben ionogenen Bestandteile wie der Zahnschmelz - verleihen diese Putzkörper den daraus hergestellten Zahnpasten eine hervorragende Reinigungs- und Polierwirkung. Da die Abrasität von DCP-A ca. 7mal höher ist als die von DCP-D ist es möglich, durch Kombination beider Putzkörper Putzkraft und Polierwirkung den jeweiligen Erfordernissen entsprechend einzustellen. Bevorzugt ist jedoch der alleinige Einsatz von DCP-D, da es den wesentlichsten Anforderungen entspricht, die an einen modernen Zahnpastenputzkörper gestellt werden. Die Beimischung von DCP-A wird vor allen Dingen dann vorgenommen, wenn hartnäckige Beläge, wie z.B. Raucherbeläge, entfernt werden sollen. Aber auch für die Formulierung von sogenannten Antiplaque-Pasten ist die Verwendung von Kombinationen von DCP-D/A vorgeschlagen und in der Praxis realisiert worden.

Die relevanten Eigenschaften von für den Einsatz in Zahnpasten geeigneten DCP-D- und DCP-A-Qualitäten sind in der einschlägigen Fach- und Patentliteratur vielfältig beschrieben worden. Es ist insbesondere die Spezifikation der Toilet Goods Association (T.G.A. Nr. 35), in der im wesentlichen die Produktmerkmale festgelegt sind, die von DCP-Produkten erfüllt werden müssen, wenn sie für die Verwendung in Zahnpasten geeignet sein sollen. Darüberhinaus sind im Laufe der Zeit für DCP-D und DCP-A erweiterte Testmethoden entwickelt worden, um spezielle, für den Einsatz in Zahnpasten relevante Eigenschaften beurteilen zu können. Teilweise sind die vorhandenen Methoden dergestalt verbessert worden, daß qualitative Befunde auch quantitativ erfaßt werden können.

Es ist auch bekannt das DCP-D, welches als Putzkörper in Zahnpasten Verwendung findet, gegen Hydrolyse und Kristallwasserverlust mit Hilfe von Dimagnesiumphsphat-trihydrat (DMP) und Natriumpyrophosphat zu stabilisieren (DE-C 26 48 061).

Dazu werden in einem Reaktor eine Calciumverbindung und Phosphorsäure unter Rühren mit einem Propellerrührer mit 2000 Upm bei einem pH-Wert zwischen 2 und 4 umgesetzt und anschließend wird auf das. entstandene PCP-D durch Umsetzung einer Magnesiumsalzlösung mit Phosphorsäure DMP niedergeschlagen. Natriumpyrophosphat kann als Stabilisator der Fällungsflotte oder dem getrockneten Produkt während der Mahlung zugesetzt werden. Nach Abtrennen des Endproduktes wird es gewaschen, getrocknet und gemahlen.

Es hat sich gezeigt, daß diese bekannten Putzkörper eine Adsorptionskraft von 40 bis 60 g $H_2O$/100 g Putzkörper aufweisen, was zur Folge hat, daß sie bei der Herstellung der Zahnpasten, je nach Menge und Art der dabei verwendeten Verdickungsmittel, in Konzentrationen von 40 bis 50 Gew% eingesetzt werden, woraus sich eine Dichte der Pasten von etwa 1,5 g/ml ergibt. Eine typische Pastenformulierung wie sie dem derzeitigen Stand der Technik entspricht, ist in der nachfolgenden Tabelle 2, Beispiel 6 aufgeführt.

Überraschenderweise wurde nun gefunden, daß bei gleicher Pastenkonsistenz, Zahnpasten eine geringere Dichte aufweisen, wenn sie als Putzkörper DCP-D mit einer Adsorptionskraft von mehr als 60 g $H_2O$/100 g Putzkörper, vorzugsweise von 100 bis 135 g $H_2O$/100 g Putzkörper enthalten.

Zweckmäßigerweise ist dieser Putzkörper mit DMP stabilisiert, so daß er aus 90 bis 98 Gew% $CaHPO_4 \bullet 2,H_2O$ Rest $MgHPO_4 \bullet 3\ H_2O$ und Natriumpyrophosphat besteht.

Ferner ist es empfehlenswert, wenn er eine Korngröße von höchstens 100 μm und eine Korngrößenverteilung von
mindestens 99,95 % < 75 μm
99,0 % < 44 μm und
50,0 % < 20 μm aufweist.

Nicht vorsehbar war ferner für den Fachmann, daß ein Putzkörper mit all den oben genannten Eigenschaften erhältlich ist, wenn man

a) in einen Reaktor, der mit einer Umpumpvorrichtung und zusätzlich mit einem hochtourigen Mischorgan, einer Leistung von mehr als 3000 Ump, vorzugsweise von 5000 - 8000 Upm, versehen ist, eine wäßrige Supension von 4 bis 30 Gew% $CaCO_3$, vorzugsweise 15 - 20 Gew% $CaCO_3$, vorlegt und diese Suspension umpumpt, wobei es sich bei dem $CaCO_3$ um ein gefälltes Produkt mit einem mittleren Korndurchmesser < 10 μm, vorzugsweise < 5 μm, handelt;

b) mit Hilfe des Mischorgans in der ständig umgepumpten Suspension eine turbulente Rührzone erzeugt;

c) direkt in diese Rührzone Phosphorsäure mit einem Gehalt von mehr als 70 Gew%, vorzugsweise von 83 - 85 Gew%, im Überschuß von maximal 10 Gew%, vorzugsweise von weniger als 5 Gew%, bezogen auf die Bildung von $CaHPO_4 \bullet 2\ H_2O$ unter Einhaltung einer Temperatur von weniger als 40° C, vorzugsweise von 22 - 30° C, einspeist;

d) nach Einspeisung der Phosphorsäure, soviel einer wäßrigen Magnesiumsalzlösung, beispielsweise einer $MgCl_2$-Lösung, der Rührzone zuführt, daß sich im Endprodukt ein Gehalt an $MgHPO_4 \bullet 3 H_2O$ von 1 bis 9,5 Gew% einstellt;

e) gleichzeitig mit oder nach Zuführung der Magnesiumsalzlösung Natronlauge, vorteilhafterweise eine solche mit einer Konzentration von 25 Gew%, zudosiert bis ein End-pH-Wert von 6,0 bis 6,9, vorteilhafterweise von 6,3 - 6,4, erreicht ist und dann

f) den insgesamt gebildeten Niederschlag von der Mutterlauge abtrennt, wäscht und bei Temperaturen unterhalb 40° C trocknet.

Gegenüber dem an sich bekannten Verfahren zur Herstellung von stabilisiertem DCP-D besteht die Verbesserung der erfindungsgemäßen Arbeitsweise darin, daß man als Calciumcarbonat ein gefälltes Produkt mit einem mittleren Korndurchmesser von maximal 20 $\mu$m einsetzt, die Suspension im Reaktor umpumpt, ferner in der umgepumpten Suspension mit einem zusätzlichen hochtourigen Mischorgan mit einer Leistung von mehr als 3000 Upm eine turbulente Rührzone erzeugt und sowohl die Phosphorsäure als auch später die Magnesiumsalzlösung direkt in diese Rührzone einträgt.

Abgesehen davon, daß ein so erhaltenes DCP-D bereits in der gewünschten Korngröße anfällt und nicht erst gemahlen werden muß, benötigt man von dem erfindungsgemäßen DCP-D nur eine Einsatzmenge von ca. 33 % anstatt von ca. 48 %, um eine Paste mit ausreichender Viskosität zu erzielen. Beispiele für entsprechende Pastenformulierungen werden im nachfolgenden gegeben. Die geringere Einsatzmenge an erfindungsgemäßem Putzkörper resultiert dementsprechend in einer niedrigeren Pastendichte (ca. 1,3 g/ml). Da aber geringere Dichte ein größeres Volumen pro Gewichtseinheit bedeutet und Zahnpasten im allgemeinen nicht nach gewicht sondern nach Volumen verkauft werden, liegt es auf der Hand, daß hiermit ein ökonomischer Vorteil verbunden ist. Die Reduzierung der Pastendichte von 1,5 auf 1,3 entspricht einem Volumenzuwachs von mehr als 15 % bezogen auf die Gewichtseinheit. Mit anderen Worten: Mit der Gewichtsmenge, mit der man bei einer Paste auf Basis konventionellen DCP-D 100 Tuben Zahnpaste erzielt, erhält man mit einer erfindungsgemäßen Pastenformulierung 115 Tuben.

Zur näheren Erläuterung der vorliegenden Erfindung sollen die nachfolgenden Beispiele dienen.

Zunächst wurden in zwei Versuchen erfindungsgemäße Putzkörper hergestellt.

Versuch I:

In einem 50-l-Doppelmantelglasreaktor, der mit einer Umpumpvorrichtung in Form einer Druckmembranpumpe und einem hochtourigen Mischorgan nach dem Rotor-Stator-Prinzip, System "Ultra-Turrax ®" der Firma Janke und Kunkel, versehen war wurde eine Suspension von 5000 g feinteiligem, gefälltem Calciumcarbonat Marke "Schäfer Cl ®" der Firma Schäfer Dietz / Lahn, mittlerer Korndurchmesser < 5 $\mu$m in 27 Liter Wasser vorgelegt und umgepumpt.

In dieser umgepumpten Suspension wurde mit dem hochtourigen Mischorgan bei einer Umdrehung von 8000 Upm eine turbulente Rührzone erzeugt und direkt in diese Rührzone 7500 g 84,5 %ige Phosphorsäure über eine Dosierpumpe zugegeben. Der Überschuß an $H_3PO_4$, bezogen auf DCP-D, betrug dabei ca. 3 Gew%. Durch Kühlung wurde die Reaktionstemperatur auf 23° C gehalten.

Nachdem die Zugabe der Phosphorsäure beendet war, wurden der Rührzone eine der Bildung von 5,2 Gew% $MgHPO_4 \bullet 3 H_2O$ äquivalente Menge einer 33 %igen $MgCl_2$-Lösung zugeführt. Anschließend wurde dem Reaktionsgemisch 25 %ige Natronlauge zugesetzt, bis ein pH-Wert zwischen 6,3 - 6,4 erreicht war. Dann wurden als weiterer Stabilisator 88 g $Na_4P_2O_7$ zugegeben.

Dannach wurde das so entstandene DCP-D I auf einer Vakuum-Nutsche von der Mutterlauge abgetrennt gewaschen und getrocknet.

Versuch II:

Es wurde im Prinzip wie in Versuch I gearbeitet. Vorgelegt und umgepumpt wurde eine Suspension von 1475 g gefälltem Calciumcarbonat Marke "SOCAL®P2" der Firma Solvay, mittlerer Kondurchmesser 4 $\mu$m, in 30 Litern Wasser.

In das Mischorgan wurden bei 5000 Upm 2000 g 84 %ige $H_3PO_4$ gegeben und danach 275 g 33 %ige $MgCl_2$-Lösung sowie 620 g 25 %ige NaOH so daß sich ein pH-Wert von 6,4 einstellt.

Erhalten wurden 2550 g DCP-D II (ca. 95 % der Theorie), das noch zusätzlich durch Vermischen mit 22 g $Na_4P_2O_7$ in bekannter Weise stabilisiert wurde.

Die für den Einsatz in Zahnpasten relevanten chemischen und physikalischen Eigenschaften der gemäß den Versuchen I und II erhaltenen Produkte DCP-D I und DCP-D II sind in Tabelle 1 aufgelistet. Zum Vergleich sind die entsprechenden Werte eines Handelsproduktes DCP-D III in der üblichen Darbietungs-

3

form und in aufgemahlenem Zustand DCP-D III (gemahlen) gegenübergestellt, um den Einfluß der Korngröße auf die Wasseradsorption zu demonstrieren. Wie ersichtlich wird durch die Aufmahlung (Verkleinerung des mittleren Korndurchmessers von 16,0 auf 7,1 μm) die Wasseradsorption nur unwesentlich, nämlich von 52 auf 58 erhöht und reicht an die Werte der erfindungsgemäßen Produkte DCP-D I und DCP-D II nicht annähernd heran, obwohl hier die mittleren Korndurchmesser mit 12,0 und 17 μm weitaus größer ausfallen.

Die Eignung eines DCP-D für die Verwendung als Zahnpastenputzkörper hängt stark von seiner Stabilität im wäßrigen Milieu ab, die dadurch gekennzeichnet ist, daß möglichst kein Kristallwasserverlust eintritt und keine hydrolytische Zersetzung unter Bildung von Hydroxylapatit, $Ca_5(PO_4)_3(OH)$, und Orthophosphorsäure stattfindet. Beide Reaktionen sind unerwünscht, weil sie die Konsistenz bzw. den pH-Wert der Zahnpaste beeinflussen, wodurch es - meist verbunden mit einer Phasentrennung - bis zur zementartigen Verhärtung der Paste kommen kann.

Die Neigung zum Kristallwasserverlust läßt sich qualitativ mit Hilfe des sogenannten Glycerin-Set-Tests (T.G.A. Nr. 35) erfassen. Dieser Test beruht darauf, daß ein Slurry, bestehend aus einem etwa gleichteiligen Gemisch aus Glycerin/Wasser und DCP-D, im siedenden Wasserbad 30 Minuten erhitzt wird. Eine ausreichende Stabilität liegt dann vor, wenn der Slurry nach dem Abkühlen noch frei fließfähig ist. Zur Quantifizierung des hierbei auftrenden Kristallwasserverlustes wird das DCP-D isoliert, gewaschen und getrocknet und der Glühverlust bei 800°C bestimmt. Der erhaltene Wert gibt Aufschluß über den aufgetretenen Kristallwasserverlust infolge der Hitzebehandlung, wenn man ihn mit dem Glühverlust des unbehandelten Produktes vergleicht. Wie der Tabelle 1 zu entnehmen ist, verhalten sich die erfindungsgemäßen Produkte besser als das Handelsprodukt.

Die Hydrolysestabilität prüft man an einer wäßrigen DCP-D-Suspension, der ca. 6,5 Gew% NaF, bezogen auf das eingesetzte DCP-D, zugegeben werden, um die Bildung von Hydroxyl- bzw. Fluorapatit zu beschleunigen. Ein DCP-D gilt dann als ausreichend stabil, wenn die wäßrige Suspension bei einer Temperatur von 60°C nach 4 Stunden den pH-Wert von 3 nicht unterschreitet. Um die stattgefundene Hydrolyse zu quantifizieren, wird die nach 4 Stunden gebildete Orthophosphorsäure alkalimetrisch erfaßt. Die diesbezüglichen Werte in Tabelle 1 weisen aus, daß die erfindungsgemäßen Produkte der Handelsware mindestens ebenbürtig sind.

Mit den erfindungsgemäßen DCP-D (I und II) und dem handelsüblichen Produkt als solchem in seiner aufgemahlenen Form (III, III gemahlen) wurden nach dem üblichen, dem Fachmann geläufigen Verfahren Zahnpasten hergestellt, deren Rezepturen in der Tabelle 2 zusammengefaßt sind.

Die für die Praxis wichtigen Eigenschaften der resultierenden Zahnpasten sind in Tabelle 3 aufgelistet.

Man erkennt, daß die Zahnpasten, hergestellt aus dem erfindungsgemäßen DCP-D (Beispiel 1-5), Dichten aufweisen, die im Gegensatz zu der Paste auf Basis der Handelsware (Beispiel 6, Dichte: 1,52 g/ml) mit 1,32 - 1,37 g/l wesentlich niedriger ausfallen.

Die Abrasivitäten, gemessen nach der Kupferabriebmethode (bezogen auf Standard-DCP-D mit willkürlichem Abrasivitätsindex = 100) liegen bei dem erfindungsgemäßen DCP-D (Beispiel 1-4) etwa um die Hälfte niedriger als bei der Handelsware (Beispiel 6).

Mit Beispiel 5 soll demonstriert werden, daß durch Beimischung kleiner Mengen von handelsüblichem wasserfreiem Dicalciumphosphat (DCP-A) die Abrasivität, wenn gewünscht, angehoben werden kann, so daß der Wert von DCP-D-Handelsware ohne weiteres erreicht werden kann.

Zur Erzielung einer ausreichenden Kariesprophylaxe ist es entscheidend, daß eine Zahnpaste eine ausreichende Fluorverträglichkeit besitzt, d.h. daß das ursprünglich eingebrachte Fluor - in DCP-Pasten üblicherweise als $Na_2PO_3F$ - in löslicher Form weitgehend erhalten bleibt. Die sogenannte Fluor-Retention wird von der Fachwelt in Abhängigkeit von der Lagerzeit jeweils bei Umgebungstemperatur (20°C) und bei erhöhter Temperatur (43°C) bestimmt. Die in der Tabelle 3 ausgewiesenen Werte für die Fluor-Retention zeigen eine eindeutige Überlegenheit der erfindungsgemäßen Zahnpasten gegenüber der konventionellen Paste.

Die unter denselben Temperatur/Zeit-Bedingungen gemessenen Viskositäten der einzelnen Beispiele zeigen normales Verhalten. Auch die visuelle Beurteilung der erfindungsgemäßen Pasten, bezüglich des äußeren Erscheinungsbildes läßt gegenüber der konventionellen Paste keinen Nachteil erkennen.

## Tabelle 1

| Chem./physikalische Eigenschaften | | DCP-D I | DCP-D II | DCP-D III | DCP-D III (gemahlen) |
|---|---|---|---|---|---|
| **Granulometrie:** | | | | | |
| a) Naßsiebung < 75 μm | [Gew.-%] | 99,98 | 99,8 | 99,98 | 99,98 |
| < 45 μm | [Gew.-%] | 99,9 | 99,8 | 99,6 | 99,9 |
| b) Coulter-Counter 90 % < | [μm] | 17,4 | 22,0 | 29,3 | 12,3 |
| 75 % < | [μm] | 13,4 | 18,8 | 20,8 | 8,4 |
| 50 % < | [μm] | 10,0 | 15,0 | 11,9 | 6,0 |
| 25 % < | [μm] | 7,4 | 10,7 | 6,3 | 4,2 |
| 10 % < | [μm] | 5,5 | 7,8 | 3,9 | 3,1 |
| Mittlerer Korndurchmesser d' | [μm] | 12,0 | 17,0 | 16,0 | 7,1 |
| Wasseradsorption | [g $H_2O$/100 g DCP-D] | 134 | 120 | 52 | 58 |
| Rüttelgewicht nach DIN 53 194 | [g/l] | 530 | 540 | 980 | 860 |
| pH-Wert (20 %ige Suspension) | | 7,5 | 7,5 | 7,4 | 7,4 |
| Glühverlust bei 800 °C | [Gew.-%] | 27,6 | 26,9 | 25,3 | 25,2 |
| Glyzerin-Set-Test (T.G.A.) | | gut | gut | gut | gut |
| Glühverlust nach Glycerin-Set-Test | | 24,5 | 24,8 | 21,0 | 20,6 |
| pH-Abfall-Test: t bis pH < 3 | [min] | > 240 | > 240 | > 240 | > 240 |
| [mg $H_3PO_4$/25 g DCP-D] in 240 min | | 550 | 560 | 530 | 540 |

EP 0 280 077 B1

## Tabelle 2

| Bestandteile | Beispiel 1 | Beispiel 2 | Beispiel 3 | Beispiel 4 | Beispiel 5 | Beispiel 6 |
|---|---|---|---|---|---|---|
| | Gewichtsteile | | | | | |
| DCP-D I | 33,0 | 33,0 | 33,0 | – | 32,0 | – |
| DCP-D II | – | – | – | 34,5 | – | – |
| DCP-D III | – | – | – | – | – | 48,0 |
| DCP-A | – | – | – | – | 2,0 | – |
| Wasser | 39,84 | 33,84 | 37,84 | 36,54 | 36,84 | 24,84 |
| Glycerin (99 %ig) | 11,0 | – | 24,0 | 24,0 | 24,0 | 11,0 |
| Sorbit (70 %ig) | 11,0 | 28,0 | – | – | – | 11,0 |
| Na-Laurylsulfat | 2,0 | 2,0 | 2,0 | 2,0 | 2,0 | 2,0 |
| Carboxymethylcellulose | 1,0 | 1,0 | 1,0 | 0,8 | 1,0 | 1,0 |
| Aroma | 1,0 | 1,0 | 1,0 | 1,0 | 1,0 | 1,0 |
| Na-Monofluorophosphat | 0,76 | 0,76 | 0,76 | 0,76 | 0,76 | 0,76 |
| Na-Saccharinat | – | – | 0,2 | – | – | – |
| Acesulfam | 0,2 | 0,2 | – | 0,2 | 0,2 | 0,2 |
| K-Sorbat | 0,2 | 0,2 | 0,2 | 0,2 | 0,2 | 0,2 |

EP 0 280 077 B1

Tabelle 3

| | Beispiel 1 | Beispiel 2 | Beispiel 3 | Beispiel 4 | Beispiel 5 | Beispiel 6 |
|---|---|---|---|---|---|---|
| Abrasivitätsindex | 50 | 60 | 55 | 45 | 100 | 115 |
| Dichte [g/ml] | 1,32 | 1,33 | 1,33 | 1,37 | 1,36 | 1,52 |
| Fluor-Retention 20 °C/43 °C [mg/kg F] | | | | | | |
| sofort | 940/- | 950/- | 925/- | 940/- | 970/- | 790/- |
| nach 30 Tagen | 890/760 | 900/780 | 910/740 | 880/820 | 950/860 | 750/470 |
| nach 90 Tagen | 820/610 | 850/700 | 790/560 | 870/760 | 940/760 | 640/280 |
| nach 180 Tagen | 800/550 | 830/640 | 740/530 | 850/690 | 870/700 | 610/140 |
| Viskosität 20 °C/43 °C [mPas · 10³] | | | | | | |
| sofort | 112/- | 114/- | 113/- | 124/- | 116/- | 126/- |
| nach 30 Tagen | 121/123 | 124/127 | 119/127 | 128/132 | 122/126 | 131/134 |
| nach 90 Tagen | 127/131 | 130/135 | 127/132 | 135/140 | 127/135 | 137/142 |
| nach 180 Tagen | 130/136 | 132/140 | 131/137 | 138/145 | 132/141 | 140/146 |

**Patentansprüche**

1.  Zahnpasten auf Basis von Dicalciumphosphat-dihydrat, dadurch gekennzeichnet, daß sie als Putzkörper einen im wesentlichen aus Dicalciumphosphat-dihydrat bestehenden Putzkörper mit einer Adsorptionskraft von mehr als 60 g $H_2O$/100 g Putzkörper enthalten.

2. Zahnpasten nach Anspruch 1, dadurch gekennzeichnet, daß sie einen Putzkörper enthalten, der aus 90 bis 98 Gew% $CaHPO_4$ • 2 $H_2O$, Rest $MgHPO_4$ • 3 $H_2O$ und Natriumpyrophosphat besteht.

3. Zahnpasten nach einem der Ansprüche 1 oder 2, dadurch gekennzeichnet, daß der Putzkörper eine maximale Korngröße von 100 $\mu$m sowie eine Korngrößenverteilung von

   mindestens 99,95 % < 75 $\mu$m
   99,0 % < 45 $\mu$m und
   50,0 % < 20 $\mu$m aufweist.

4. Putzkörper auf Basis Dicalciumphosphat-dihydrat für Zahnpasten, dadurch gekennzeichnet, daß er eine Adsorptionskraft von mehr als 60 g $H_2O$/100 g Putzkörper aufweist.

5. Putzkörper nach Anspruch 4, dadurch gekennzeichnet, daß er aus 90 bis 98 Gew% $CaHPO_4$ • 2 $H_2O$, Rest Mg $HPO_4$ • 3 $H_2O$ und Natriumpyrophosphat besteht.

6. Putzkörper nach einem der Ansprüche 4 oder 5, dadurch gekennzeichnet, daß er eine Korngröße von höchstens 100 $\mu$m und eine Korngrößenverteilung von

   mindestens 99,95 % < 75 $\mu$m
   99,0 % < 45 $\mu$m und
   50,0 % < 20 $\mu$m aufweist.

7. Putzkörper nach einem der Ansprüche 4 bis 6, erhältlich, indem man

   a) in einem Reaktor, der mit einer Umpumpvorrichtung und zusätzlich mit einem hochtourigen Mischorgan, einer Leistung von mehr als 3000 Upm versehen ist, eine wäßrige Suspension von 4 bis 30 Gew% $CaCO_3$ vorlegt und diese Suspension umpumpt, wobei es sich bei dem $CaCO_3$ um ein gefälltes Produkt mit einem mittleren Korndurchmesser < 10 $\mu$m handelt;
   b) mit Hilfe des Mischorgans in der ständig umgepumpten Suspension eine turbulente Rührzone erzeugt;
   c) direkt in diese Rührzone Phosphorsäure mit einem Gehalt von mehr als 70 Gew% im Überschuß von maximal 10 Gew%, bezogen auf die Bildung von $CaHPO_4$ • 2 $H_2O$ unter Einhaltung einer Temperatur von weniger als 40° C, einspeist;
   d) nach Einspeisung der Phosphorsäure, soviel einer wäßrigen Magnesiumsalzlösung der Rührzone zuführt, daß sich im Endprodukt ein Gehalt an $MgHPO_4$ • 3 $H_2O$ von 1 bis 9,5 Gew% einstellt;
   e) gleichzeitig mit oder nach Zuführung der Magnesiumsalzlösung Natronlauge zudosiert bis ein End-pH-Wert von 6,0 bis 6,0 erreicht ist und
   f) den insgesamt gebildeten Niederschlag von der Mutterlauge abtrennt, wäscht und bei Temperaturen unterhalb 40° C trocknet.

8. Verfahren zur Herstellung eines mit Dimagnesiumphosphat-trihydrat stabilisierten Putzkörpers aus Dicalciumphosphat-dihydrat, indem man in einem Reaktor eine wäßrige Suspension von Calciumcarbonat mit mindestens einer zur quantitativen Bildung von Dicalciumphosphat-dihydrat ausreichenden Menge Phosphorsäure vermischt und umsetzt, anschließend durch Umsetzung einer wäßrigen Magnesiumsalzlösung mit einer zur Bildung von Dimagnesiumphosphat-trihydrat ausreichenden Menge Phosphorsäure und Zugabe von Natronlauge bis zu einem pH-Wert von 6,9 auf das gebildete Dicalciumphosphat-dihydrat Dimagnesiumphosphat-trihydrat in einer Menge von 2 bis 4 Gew%, bezogen auf das Dicalciumphosphat-dihydrat, niederschlägt, dann das gesamte Reaktionsprodukt von der Mutterlauge abtrennt, wäscht und bei Temperaturen unter 40° C schonend trocknet, dadurch gekennzeichnet, daß man als Calciumcarbonat ein gefälltes Calciumcarbonat mit einem mittleren Korndurchmesser von maximal 20 $\mu$m einsetzt, die Calciumcarbonatsuspension im Reaktor umpumpt, zusätzlich in der umgepumpten Suspension mit einem hochtourigen Mischorgan mit einer Leistung von mehr als 3000 Upm eine turbulente Rührzone erzeugt und sowohl die Phosphorsäure als auch später die Magnesiumsalzlösung direkt in diese Rührzone einträgt.

EP 0 280 077 B1

**Claims**

1. A toothpaste based on dicalcium phosphate dihydrate, which contains, as the cleaning substance, a cleaning substance which essentially consists of dicalcium phosphate dihydrate and has an adsorption power of more than 60 g of $H_2O$/100 g of cleaning substance.

2. A toothpaste as claimed in claim 1, which contains a cleaning substance which consists of 90 to 98 % by weight of $CaHPO_4 \cdot 2H_2O$, the remainder being $MgHPO_4 \cdot 3H_2O$ and sodium pyrophosphate.

3. A toothpaste as claimed in one of claims 1 or 2, in which the cleaning substance has a maximum particle size of 100 $\mu$m and a particle size distribution of

   at least 99.95 % < 75 $\mu$m,
   99.0 % < 45 $\mu$m and
   50.0 % < 20 $\mu$m.

4. A cleaning substance based on dicalcium phosphate dihydrate for toothpastes, which has an adsorption power of more than 60 g of $H_2O$/100 g of cleaning substance.

5. A cleaning substance as claimed in claim 4, which consists of 90 to 98 % by weight of $CaHPO_4 \cdot 2H_2O$, the remainder being $MgHPO_4 \cdot 3H_2O$ and sodium pyrophosphate.

6. A cleaning substance as claimed in one of claims 4 or 5, which has a particle size of not more than 100 $\mu$m and a particle size distribution of

   at least 99.95 % < 75 $\mu$m,
   99.0 % < 45 $\mu$m and
   50.0 % < 20 $\mu$m.

7. A cleaning substance as claimed in one of claims 4 to 6, obtainable by

   a) initially introducing an aqueous suspension of 4 to 30 % by weight of $CaCO_3$ into a reactor provided with a circulation pump device and additionally with a high-speed mixing organ having a speed of more than 3000 revolutions per minute, and circulating this suspension, the $CaCO_3$ being a precipitated product having an average particle diameter of < 10 $\mu$m;
   b) generating a turbulent stirring zone, with the aid of the mixing organ, in the constantly circulated suspension;
   c) feeding phosphoric acid having a content of more than 70 % by weight directly into this stirring zone in an excess of not more than 10 % by weight, based on the formation of $CaHPO_4 \cdot 2H_2O$, a temperature of less than 40 $^\circ$C being maintained;
   d) after feeding in the phosphoric acid, adding an aqueous magnesium salt solution to the stirring zone in an amount such that a content of $MgHPO_4 \cdot 3H_2O$ of 1 to 9.5 % by weight is established in the end product;
   e) at the same time as or after the addition of the magnesium salt solution, metering in sodium hydroxide solution until a final pH of 6.0 to 6.9 is reached and
   f) separating off the total precipitate formed from the mother liquor, and washing the precipitate and drying it at temperatures below 40 $^\circ$C.

8. A process for the preparation of a dicalcium phosphate dihydrate cleaning substance stabilized with dimagnesium phosphate trihydrate by mixing and reacting an aqueous suspension of calcium carbonate with at least an amount of phosphoric acid sufficient for quantitative formation of dicalcium phosphate dihydrate in a reactor, subsequently reacting an aqueous magnesium salt solution with an amount of phosphoric acid sufficient to form dimagnesium phosphate trihydrate and adding sodium hydroxide solution up to a pH of 6.9 to precipitate dimagnesium phosphate trihydrate, in an amount of 2 to 4 % by weight, based on the dicalcium phosphate dihydrate, onto the dicalcium phosphate dihydrate formed, thereafter separating off the entire reaction product from the mother liquor and washing the product and gently drying it at temperatures below 40 $^\circ$C, which comprises using a precipitated calcium carbonate having an average particle diameter of not more than 20 $\mu$m as the calcium carbonate,

9

circulating the calcium carbonate suspension in the reactor, additionally generating a turbulent stirring zone, using a high-speed mixing organ with a speed of more than 3000 revolutions per minute, in the circulated suspension and introducing both the phosphoric acid and also later the magnesium salt solution directly into this stirring zone.

**Revendications**

1. Pâtes dentifrices à base de phosphate dicalcique dihydraté, caractérisées en ce qu'elles contiennent comme nettoyant un nettoyant composé essentiellement de phosphate dicalcique dihydraté, ayant une force d'adsorption supérieure à 60 g de $H_2O$ par 100 g de nettoyant.

2. Pâtes dentifrices selon la revendication 1, caractérisées en ce qu'elles contiennent un nettoyant qui est composé de 90 à 98 % en poids de $CaHPO_4 \cdot 2H_2O$ et le reste de $MgHPO_4 \cdot 3H_2O$ et de pyrophosphate sodique.

3. Pâtes dentifrices selon l'une des revendications 1 ou 2, caractérisées en ce que le nettoyant présente une granulométrie maximale de 100 $\mu$m et une composition granulométrique
d'au moins 99,95 % < 75 $\mu$m
99,0 % < 45 $\mu$m et
50,0 % < 20 $\mu$m.

4. Nettoyant à base de phosphate dicalcique dihydraté pour pâtes dentifrices, caractérisé en ce qu'il présente une force d'adsorption supérieure à 60 g de $H_2O$ par 100 g de nettoyant.

5. Nettoyant selon la revendication 4, caractérisé en ce qu'il est composé de 90 à 98 % en poids de $CaHPO_4 \cdot 2H_2O$ et le reste de $MgHPO_4 \cdot 3H_2O$ et de pyrophosphate sodique.

6. Nettoyant selon l'une des revendications 4 ou 5, caractérisé en ce qu'il présente une granulométrie maximale de 100 $\mu$m et une composition granulométrique
d'au moins 99,95 % < 75 $\mu$m
99,0 % < 45 $\mu$m et
50,0 % < 20 $\mu$m.

7. Nettoyant selon l'une des revendications 4 à 6, obtenu lorsqu'on
a) introduit dans un réacteur muni d'un dispositif de transvasement par pompage et additionnellement d'un mélangeur grande vitesse, de puissance supérieure à 3 000 tr/min, une suspension aqueuse de 4 à 30 % en poids de $CaCO_3$ et qu'on transvase cette suspension, le $CaCO_3$ étant un produit précipité de diamètre de grain moyen < 10 $\mu$m;
b) crée une zone d'agitation à turbulences dans la suspension constamment transvasée, à l'aide du mélangeur;
c) introduit directement dans cette zone d'agitation de l'acide phosphorique de teneur supérieure à 70 % en poids en excès d'un maximum de 10 % en poids, par rapport à la formation de $CaHPO_4 \cdot 2H_2O$, en conservant une température inférieure à 40°C;
d) amène à la zone d'agitation, après introduction de l'acide phosphorique, une quantité d'une solution aqueuse de sel de magnésium suffisante pour obtenir dans le produit final une teneur en $MgHPO_4 \cdot 3H_2O$ de 1 à 9,5 % en poids;
e) ajoute en même temps ou après l'apport de la solution de sel de magnésium une solution d'hydroxyde de sodium jusqu'à ce que l'on obtienne un pH final compris entre 6,0 et 6,9; et
f) sépare de la liqueur mère le précipité formé au total, qu'on le lave et qu'on le sèche à des températures inférieures à 40°C.

8. Procédé de fabrication d'un nettoyant en phosphate dicalcique dihydraté, stabilisé par du phosphate de dimagnésium trihydraté, dans lequel on mélange et met à réagir dans un réacteur une suspension aqueuse de carbonate de calcium avec au moins une quantité d'acide phosphorique suffisante pour la formation quantitative de phosphate dicalcique dihydraté, qu'on précipite ensuite sur le phosphate dicalcique dihydraté formé du phosphate de dimagnésium trihydraté, en une quantité de 2 à 4 % en poids, par rapport au phosphate dicalcique dihydraté, par mise à réagir d'une solution aqueuse de sel de magnésium avec une quantité suffisante d'acide phosphorique pour la formation de phosphate de dimagnésium trihydraté et ajout d'une solution d'hydroxyde de sodium jusqu'à l'obtention d'un pH de 6,9, puis qu'on sépare le produit réactionnel total de la liqueur mère, qu'on le lave et qu'on le sèche doucement à des températures inférierures à 40° C, caractérisé en ce qu'on utilise comme carbonate de calcium un carbonate de calcium précipité, de diamètre de grain moyen maximal de 20 µm, qu'on transvase la suspension de carbonate de calcium se trouvant dans le réacteur, qu'on crée additionnellement dans la suspension transvasée une zone d'agitation à turbulence à l'aide d'un mélangeur grande vitesse de puissance supérieure à 3 000 tr/min et qu'on introduit directement dans cette zone d'agitation l'acide phosphorique de même que la solution de sel de magnésium, ultérieurement.